# EUROPÄISCHE PATENTSCHRIFT

(11) **EP 2 421 487 B1**
(45) Veröffentlichungstag und Bekanntmachung des Hinweises auf die Patenterteilung: **15.05.2013**
(21) Anmeldenummer: 10722919.7
(22) Anmeldetag: 20.04.2010
(51) Int. Cl.: A61F 5/01

(54) **STRUKTURELEMENT FÜR EINE ORTHOPÄDIETECHNISCHE EINRICHTUNG**
STRUCTURAL ELEMENT FOR AN ORTHOPEDIC DEVICE
ELÉMENT DE STRUCTURE POUR UN DISPOSITIF ORTHOPÉDIQUE

(30) Priorität: 22.04.2009 DE 102009018179
(43) Veröffentlichungstag der Anmeldung: 29.02.2012
(73) Patentinhaber: Otto Bock HealthCare GmbH, 37115 Duderstadt (DE)
(72) Erfinder: JARJOUR, Wissam, 15907 Lübben (DE); KROLL-ORYWAHL, Olaf, 37083 Göttingen (DE); SCHILLING, Matthias, 37345 Weißenborn-Lüderode (DE)
(74) Vertreter: Stornebel, Kai
(86) Internationale Anmeldenummer: PCT/DE2010/000457
(87) Internationale Veröffentlichungsnummer: WO 2010/121603

(56) Entgegenhaltungen:
- WO-A1-2007/107150
- DE-A1- 10 201 134
- US-A- 5 840 047

## Beschreibung

Die Erfindung betrifft ein Strukturelement für eine orthopädietechnische Einrichtung, insbesondere für eine Orthese oder eine Prothese, das zumindest einen in dem Strukturelement integrierten Sensor aufweist, der mit Leitern zur Übertragung von Energie und/oder Sensorsignalen verbunden ist und eine orthopädische Einrichtung mit einem solchen Strukturelement. Das Dokument US 5,840,047 A stellt der Nächste Stand der Technik dar.

Bei orthopädietechnischen Einrichtungen besteht mitunter die Notwendigkeit, mehrere Komponenten miteinander zu verbinden. Eine distale Komponente kann mit einer proximalen Komponente federnd miteinander verbunden sein, beispielsweise ein Knieteil und ein Fußteil einer Knie-Knöchel-Fuß-Orthese.

Die Verwendung einer Balkenfeder aus einem faserverstärkten Verbundwerkstoff ist im Zusammenhang mit einer Fußheberorthese aus der DE 10 2004 027 252 A1 bekannt. An der gebogen ausgebildeten Balkenfeder sind Befestigungseinrichtungen zur Festlegung der Feder an dem Fuß und an dem Unterschenkel angeordnet.

Die Befestigung eines Fußteils einer Knie-Knöchel-Fuß-Orthese an einem Unterschenkel ist aus der WO2005/05821 A2 bekannt. Das Unterschenkelteil wird dabei aus zwei zueinander beabstandet gehaltenen Metallstreben gebildet. An dem distalen Ende einer jeden Metallstrebe ist ein Schwenkbolzen angeordnet, über den ein Fußteil schwenkbar mit den Streben verbunden ist.

Die US 2008/0039756 A1 beschreibt eine Knie-Knöchel-Fuß-Orthese, bei der das Unterschenkelteil durch einen starren Rahmen ausgebildet ist, an dem über ein Fußgelenk ein Fußteil zur Abstützung eines Fußes angeordnet ist. Über einen Aktuator kann das Fußteil bewegt werden.

Neben einer Anwendung in einer Knie-Knöchel-Fuß-Orthese können die Strukturelemente auch in Prothesen für die unteren oder oberen Extremitäten sowie für Orthesen im Rumpfbereich oder an oberen Extremitäten eingesetzt werden.

Um über die in dem Strukturelement auftretenden oder die durch das Strukturelement übertragenen Kräfte Informationen zu erhalten, besteht die Möglichkeit, dass an oder in dem Strukturelement ein Sensor angeordnet ist, der die Sensordaten an einen Empfänger ausgibt. Neben einer drahtlosen Übertragung ist üblicherweise vorgesehen, dass Leiter zur Übertragung von Energie und/oder Sensorsignalen physisch mit dem Sensor verbunden sind und aus dem Strukturelement herausgeführt werden müssen. An dem Ende dieser Leiter sind in der Regel Steckkontakte angeordnet, um nach dem Einbau des Strukturelementes die Sensordaten auslesen zu können.

Die US 5,840,047 A betrifft eine Sensoreinrichtung zur Überwachung einer relativen Position einer Protheseneinrichtung zu einem Stumpf, wobei die Protheseneinrichtung an dem Stumpf angeordnet ist. Der Sensor ist in einem Schaft angeordnet, in dem der Stumpf aufgenommen ist. Über ein Kabel ist der Sensor mit einer Batterie und einer Alarmeinrichtung verbunden.

Aufgabe der vorliegenden Erfindung ist es, ein längenanpassbares Strukturelement bereitzustellen, das an oder in entsprechenden Aufnahmeeinrichtungen orthopädietechnischer Komponenten eingesetzt werden kann, wobei die Länge des Strukturelementes an die individuellen Gegebenheiten des Nutzers der orthopädietechnischen Einrichtung anpassbar ist.

Erfindungsgemäß wird diese Aufgabe durch ein Strukturelement mit den Merkmalen des Hauptanspruches gelöst. Vorteilhafte Ausgestaltungen und Weiterbildungen der Erfindung sind in den Unteransprüchen aufgeführt. Das erfindungsgemäße Strukturelement für eine orthopädietechnische Einrichtung, insbesondere für eine Orthese oder eine Prothese, das zumindest einen in oder an dem Strukturelement angeordneten, z.B. integrierten Sensor aufweist, der mit Leitern zur Übertragung von Energie und/oder Sensorsignalen verbunden ist, sieht vor, dass die Leiter in dem Strukturelement integriert sind und sich entlang zumindest eines Endbereiches des Strukturelementes erstrecken. Die Leiter münden in dem Endbereich und bilden dort Kontaktflächen aus, über die das Sensorsignal abgenommen oder Energie zu- bzw. abgeführt werden kann. Da sich die Kontaktflächen über eine gewisse Längserstreckung entlang dem Außenbereich erstrecken oder im Stirnbereich ausgebildet sind, kann im Bereich der Kontaktflächen auf eine einfache Art und Weise eine Längenanpassung durch einfaches Entfernen der überschüssigen Länge des Strukturelementes erfolgen. Die Kontaktflächen bleiben dabei in ihrer räumlichen Zuordnung unverändert, so dass die korrespondierend angeordneten Kontakte an den an das Strukturelement anzuordnenden Komponenten unverändert bleiben können.

Bevorzugt ist das Strukturelement als ein Federelement ausgebildet, beispielsweise als Balkenfeder, um zwei Komponenten unter Zulassung einer geringen federnden Verlagerung zueinander miteinander zu verbinden.

Der Sensor ist bevorzugt vollständig in dem Strukturelement eingebettet, um eine gute mechanische Abschirmung zu gewährleisten. Darüber hinaus kann der Sensor in der Nähe der neutralen Phase angeordnet werden, um den Grad der Verformung möglichst gering zu halten, wenn dies gewünscht wird. Grundsätzlich ist es möglich, dass der Sensor an der Oberfläche des Strukturelementes angeordnet ist oder nur teilweise in das Strukturelement eingebettet ist, wenn die Art des Einsatzes des orthopädietechnischen Gerätes und die Struktur des Sensors dies zulassen.

Eine Weiterbildung der Erfindung sieht vor, dass der Sensor als ein Dehnmessstreifen oder als ein Piezo-Element ausgebildet ist, wobei das Piezo-Element auch zur Erzeugung von elektrischer Energie eingesetzt werden kann.

Die Leiter sind innerhalb des Strukturelementes isoliert und bevorzugt auch in dem Mündungsbereich gegeneinander isoliert, so dass eine eindeutige Kontaktierung und Signalübertragung erfolgen kann.

In dem Endbereich können die Leiter als Schleifkontakte oder als Kontaktbahnen ausgeführt sein, die sich an zumindest einer Außenseite des Endbereiches erstrecken. Über die Kontaktbahnen oder Schleifkontakte ist es möglich, dass über einen langen Bereich eine Kontaktierung möglich ist. Die Herstellung des elektrischen Kontaktes zu den zu den Kontaktflächen korrespondierenden Kontakten ist sehr einfach, die Länge des Strukturelementes kann durch Kürzen im Bereich der Kontaktbahnen leicht realisiert werden, die mechanischen Eigenschaften des Strukturelementes werden durch die Anordnung der Leiter als Kontaktbahnen auf der Außenseite nicht oder nur geringfügig beeinträchtigt.

Alternativ oder ergänzend dazu ist es möglich, dass die Leiter stirnseitig aus dem Strukturelement herausgeführt sind und dort münden. Die stirnseitigen Enden der Leiter bilden einen stirnseitigen Abschluss der Leiter und damit die Kontaktfläche für korrespondierende Kontakte in dem Strukturelement, so dass sich die Anordnung der Kontaktflächen auch bei einer Längenveränderung des Strukturelementes nicht verändert.

Die Leiter können rohrförmig ausgebildet sein, was insbesondere bei einer stirnseitigen Herausführung der Leiter vorteilhaft ist, da diese dann gleichzeitig als Steckkontakte ausgebildet sein können und die Stecker aufnehmen können.

Ebenfalls ist es vorgesehen, dass das Strukturelement zumindest bereichsweise segmentiert ist und die Segmente gegeneinander isoliert ausgebildet sind. Dadurch ist es möglich, dass die Segmente als Leiter ausgebildet sein können und die Sensorsignalübertragung oder Energieübertragung separat voneinander ausüben können. Das Strukturelement bildet die Leiter selbst aus. Vorteilhafterweise ist das Strukturelement an seinem Umfang elektrisch isoliert ausgebildet, insbesondere wenn die Segmente als Leiter ausgebildet sind. Die Kontaktflächen können im ursprünglichen Zustand ebenfalls isoliert sein, wobei die Isolierung nach dem Kürzen im Bereich der elektrischen Kontaktierung entfernt werden kann.

Durch die Ausbildung eines eckigen Querschnittes im Strukturelement ist es möglich, einerseits eine Verdrehsicherung des Strukturelementes an dem Komponenten zu gewährleisten und andererseits ausreichend glatte Flächen bereitzustellen, an denen die Kontaktflächen ausgebildet werden können.

Das Strukturelement kann aus einem Karbonfaserkomposit, einem Glasfaserkomposit und/oder einem Kunststoff hergestellt sein. Diese Werkstoffe ermöglichen eine hohe Festigkeit bei geringem Gewicht und einer ggf. gewünschten elektrischen Leitfähigkeit und Federwirkung. Neben einer Ausgestaltung der Leiter als elektrische Leiter ist vorgesehen, dass die Leiter als Lichtleiter ausgebildet sind. Die Anordnung der Leiter ist dabei von untergeordneter Bedeutung, ebenso ist die Art der Messung nicht für die Leiterführung in oder an dem Strukturelement entscheidend; zu Zwecken der Potentialtrennung kann es sinnvoll sein, das Signal oder Teile des Signals optisch auszukoppeln.

Die Sensoren oder Sensoreinrichtungen erfassen die Belastungen innerhalb des Strukturelementes oder die Veränderungen in der Struktur z.B. auf der Grundlage von Widerstandsänderungen oder über elektrische Impulse, die sich in Abhängigkeit von der Belastung verändern oder die durch eine Belastung erzeugt werden. Dehnmessstreifen, optische Dehnmessstreifen oder auf dem Piezo-Effekt basierende Sensoren können an oder in dem Strukturelement angeordnet sein. Eine Variante des Sensors sieht vor, dass eine Lichtquelle mit einem speziell ausgebildeten Lichtleiter verbunden ist, der in dem Strukturteil eingelassen oder darin eingebettet ist. Die Lichtquelle sendet Lichtsignale durch den oder die Lichtleiter, wobei während der Belastung des Strukturteils die Lichtsignale verändert werden, indem der oder die Lichtleiter die Signale unterschiedlich reflektieren. Aus der Art und dem Umfang der Abweichung der Reflexion kann dann auf die Art und den Umfang der Belastung oder Verformung des Strukturelementes geschlossen werden. In den Endbereichen des Strukturelementes kann der Lichtleiter eine Struktur aufweisen, die bei einer Verformung keine Veränderungen des Lichtsignals induziert, dies ist jedoch nicht unbedingt notwendig. Die Lichtleiter können durch gezielt eingebrachte Faserbeschädigungen oder durch das Brennen von Absorptionsgittern selbst den Sensor ausbilden.

Die orthopädietechnische Einrichtung sieht vor, dass ein Oberteil und ein Unterteil mit einem wie oben beschriebenen Strukturelement miteinander verbunden sind. An dem Oberteil und/oder dem Unterteil können dabei Aufnahmeeinrichtungen zur Festlegung des Strukturelementes angeordnet sein, in denen Kontakte angeordnet sind, die korrespondierend zu den Kontaktflächen des Strukturelementes im montierten Zustand ausgebildet sind. Dadurch ist es möglich, das Strukturelement einfach in die Aufnahmeeinrichtungen einzustecken und dort zu fixieren, beispielsweise zu verschrauben oder durch Klemmeinrichtungen dort sicher zu lagern.

Die Kontakte und/oder die Kontaktflächen können in Richtung aufeinander zu federbelastet und verlagerbar ausgebildet sein, insbesondere sind die Kontakte in den Aufnahmeeinrichtungen federbelastet, um auch bei einer nicht zu vermeidenden Ungenauigkeit bei der Kürzung des Strukturelementes einen Längenausgleich und eine sichere Kontaktierung bereitstellen zu können. Dadurch ist es möglich, dass das Strukturelement mit einem gewissen Spiel innerhalb der Aufnahmeeinrichtungen festgelegt werden kann. Grundsätzlich ist es jedoch auch möglich, dass die Kontaktflächen in Richtung auf die Kontakte verlagerbar und federbelastet ausgebildet sind, während die Kontakte ortsfest ausgebildet sind.

Die Kontakte können als Steckkontakte, Schraubkontakte oder Klemmeinrichtungen ausgebildet sein, ebenfalls kann der Kontakt mit einer eine Isolierung durchdringenden Spitze versehen sein, so dass die ursprünglich vorhandene vollständige Isolierung durch die Montage durchbrochen und ein sicherer Kontakt nur an der dafür vorgesehenen Stelle realisiert wird.

Nachfolgend werden Ausführungsbeispiele der Erfindung anhand der Figuren näher erläutert. Es zeigen:
- Figuren 1a und 1b: - Ansichten eines Strukturelementes mit Kontaktflächen an der Außenseite;
- Figuren 2a und 2b: - Ansichten eines Strukturelementes mit stirnseitig mündenden Leitern;
- Figuren 3a bis 3c: - Ansichten eines Strukturelementes mit integrierten Leitersegmenten;
- Figuren 4a bis 4c: - Ansichten eines Strukturelementes mit einer Kombination aus einem Lichtleiter und einem Segmentleiter; sowie
- Figur 5: - eine Anwendung eines Strukturelementes in einer Orthese.

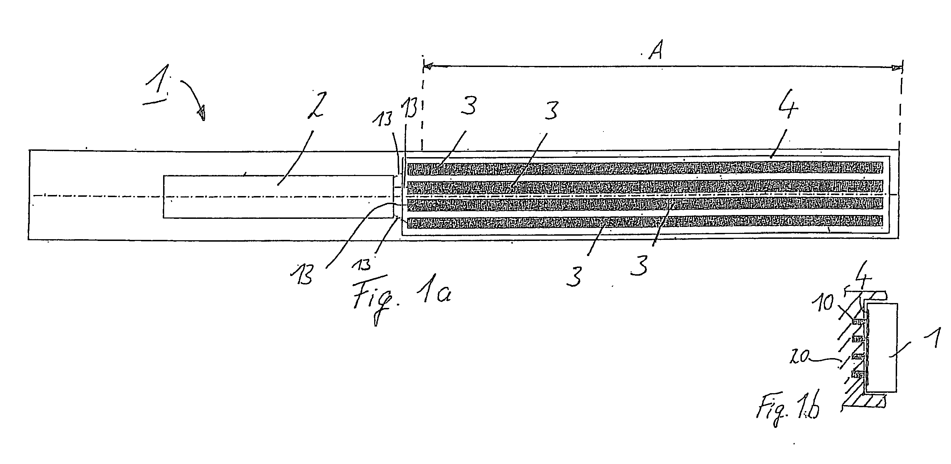

In der Figur 1a ist in einer schematischen Seitenansicht ein Strukturelement 1 aus einem Kunststoff, einem Faserverbundwerkstoff oder dergleichen gezeigt. Das Strukturelement 1 weist eine langgestreckte, balkenartige Form auf und hat einen im Wesentlichen rechtwinkligen Querschnitt, was in der Figur 1b zu sehen ist. In der dargestellten Ausführungsform bildet das Strukturelement 1 gleichzeitig ein Federelement.

In dem Strukturelement 1 ist ein Sensor 2 integriert, beispielsweise eingegossen, eingespritzt oder einlaminiert. Das Sensorelement 2 ist beispielsweise als ein Piezoelement, Drucksensor oder Dehnmessstreifen ausgebildet. Auch andere Sensoren können innerhalb des Strukturelementes 1 angeordnet sein, ebenfalls können mehrere Sensoren in einem Strukturelement 1 eingebaut sein.

Von dem Sensorelement 2 zweigen Leiter 13 ab, die die innerhalb des Sensors 2 erzeugten Signale weiterleiten. In der in Figur 1 dargestellten Ausführungsform sind die Leiter 13 als elektrische Leiter ausgebildet und mit einer Platine 4 kontaktiert, die an einer Seitenfläche des Strukturelementes 1 angeordnet ist. Die Leiter 13 sind mit den auf der Platine 4 ausgebildeten Kontaktflächen 3 verbunden, die auf der nach außen gerichteten Oberfläche der Platine 4 angeordnet sind. Die Kontaktflächen 3 und die Platine 4 erstrecken sich entlang eines Endbereiches A des Strukturelementes 1, der individuell kürzbar ist. Über die Länge des Endbereiches A kann die Gesamtlänge des Strukturelementes 1 eingestellt werden, so dass die Länge der federnden Verbindung zwischen einem Oberteil und einem Unterteil bzw. zwischen zwei orthopädietechnischen Komponenten eingestellt werden kann. Dadurch ist es möglich, bei gleichbleibenden Ober- und Unterteilen eine einfache Anpassung an die individuellen Bedürfnisse eines Patienten oder eines Nutzers einer orthopädietechnischen Einrichtung zu ermöglichen. Ebenfalls kann durch eine Längenveränderung des Strukturelementes 1 die Steifigkeit der gesamten orthopädietechnischen Einrichtung verändert werden.

In dem Ausführungsbeispiel gemäß Figur 1a bilden die Kontaktflächen 3 auf der Außenseite Leiterbahnen, die als eine Art Schleifkontakte ausgebildet sind. Die einzelnen Kontaktflächen 3 sind gegeneinander elektrisch isoliert und räumlich voneinander beabstandet, so dass die über die Kontaktflächen 3 abgeleiteten Signale durch von außen aufgebrachte Kontakte 10, die in der Figur 1b dargestellt sind, abgenommen werden können. Die Kontakte 10 sind in einer nur teilweise dargestellten Aufnahme 20 angeordnet, in die das Strukturelement 1 eingeführt und in der es fixiert wird. Das gesamte Strukturelement 1 kann im ursprünglichen Zustand außen mit einer elektrischen Isolierung versehen sein, die erst nach dem Kürzen auf die gewünschte Länge in dem Bereich der Kontaktierung durch die Kontakte 10 in dem Oberteil und/oder in dem Unterteil entfernt wird. Dieses Entfernen der Isolierung kann entweder separat in einer gesonderten Verrichtung erfolgen oder über die Kontakte 10 selbst, die die Isolierung bei der Montage entweder entfernen oder durchdringen. Dazu ist es vorteilhaft, wenn die Kontakte 10 scharfkantig oder spitz ausgebildet sind, ebenfalls können die Kontakte 10 als Schraubkontakte ausgebildet sein, die in Richtung auf die Kontaktflächen 3 verlagerbar gelagert sind. Durch die Anordnung der Kontaktflächen außen an dem Endbereich des Strukturelementes 10 ist es möglich, eine individuelle Anpassung der Länge des Strukturelementes zu erreichen, ohne dass Kabel mit daran befestigten Steckern oder Steckeraufnahmen gekürzt werden müssen. Weiterhin wird keine gesonderte Steckverbindung benötigt und es entfällt die Notwendigkeit, freie Kabelenden oder freie Kabellängen unterzubringen und fixieren zu müssen. Insbesondere bei Orthesen oder Prothesen stören freiliegende Kabel bei der Benutzung und stellen ein Gefahrenpotenzial dar, da offene Steckverbindungen und Kabelverbindungen gemäß dem Stand der Technik im täglichen Betrieb getrennt oder zerstört werden können.

In der Draufsicht gemäß Figur 1b ist zu erkennen, dass die abnehmenden Kontakte 10 von außen auf die Kontaktflächen 3 aufgesetzt werden. Wird das Strukturelement 1 mit seinem Endbereich A, der auf seine gewünschte Länge gekürzt wurde, in eine Aufnahmeeinrichtung eingeführt, erfolgt dies meist durch Einschieben, so dass die Kontakte 10 auf den Kontaktflächen 3 entlang gleiten und einen guten elektrischen Kontakt bereitstellen. Die Kontakte 10 können in Richtung auf die Kontaktflächen 3 federbelastet und elastisch gelagert sein, so dass fertigungsbedingte Toleranzen leicht ausgeglichen werden können und darüber hinaus ein sicheres Andrücken der Kontakte 10 an die Kontaktflächen 3 gewährleistet ist.

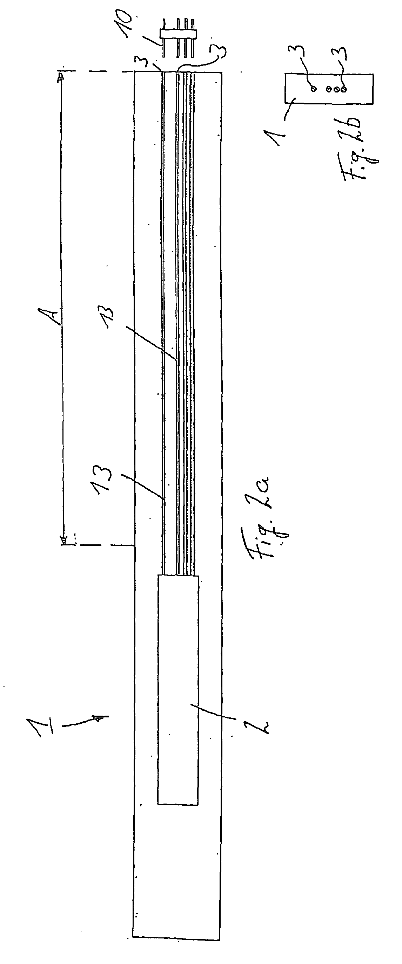

In der Figur 2 ist eine Variante der Erfindung dargestellt. Die Figur 2a zeigt das Strukturelement 1 mit dem integrierten Sensor in einer schematischen Seitenansicht. Von dem Sensor 2 erstrecken sich Leiter 13 durch das Strukturelement 1 in Richtung auf ein stirnseitiges Ende und münden dort in Gestalt von Kontaktflächen 3 innerhalb des Querschnittes des Strukturelementes 1, was in der Figur 2b zu erkennen ist. Die Leiter 13 sind als Hohlprofile oder Röhrchen ausgebildet, die sich über den gesamten Endbereich A erstrecken. Korrespondierend zu den Kontaktflächen 3 sind Kontakte 10 an einem Stecker ausgebildet, die an einer Aufnahmeeinrichtung für das Strukturelement 1 angeordnet und befestigt sind. Diese Kontakte 10 können in die Leiterrohre 13 eingeführt werden, so dass eine elektrische Kontaktierung und eine sichere Verriegelung auf eine einfache Art und Weise stattfinden kann. Neben einem Einführen von stiftartigen Kontakten 10 in die Leiter 13 ist es möglich, dass die Leiter 13 als Vollprofil ausgebildet sind und ein aufliegender elektrischer Kontakt die Sensorsignale oder elektrischen Signale von dem Sensor zu den Kontakten 10 überträgt. Die Kontakte 10 können verlagerbar und federbelastet in Richtung auf die Kontaktflächen 3 ausgebildet sein. Die Leiter 13 und die Kontaktflächen 3 sind gegeneinander elektrisch isoliert. Durch Kürzen des Endbereiches A ist es möglich, die Gesamtlänge des Strukturelementes 1, das auch hier als Balkenfeder ausgebildet ist, individuell zu variieren, ohne dass eine Veränderung in der Zuordnung oder in der Übertragungsleitung stattfindet. Die Isolierung der Leiter 13 und der Kontaktflächen 3 erfolgt auch gegenüber dem Material des Strukturelementes 1, weil dieses auch elektrisch leitend sein kann.

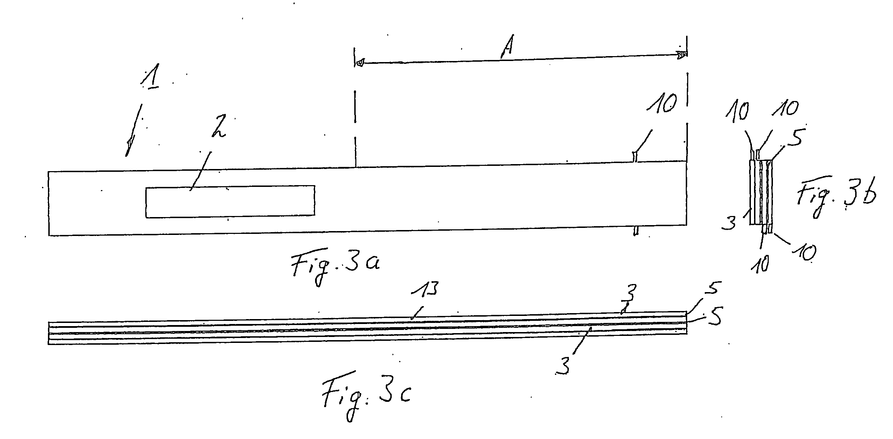

Eine weitere Variante der Erfindung sieht vor, dass das Strukturelement 1 segmentiert ist. Dabei sind die Leiter 13 als Teile des Strukturelementes 1 ausgebildet und übernehmen kraftübertragende Aufgaben. Eine solche Variante ist in den Figuren 3a bis 3c dargestellt, die Figur 3a zeigt eine schematische Draufsicht, die Figur 3b eine stirnseitige Ansicht und die Figur 3c eine Seitenansicht des Strukturelementes 1. Der Sensor 2 ist wie in den Figuren 1 und 2 beschrieben in dem Strukturelement 1 eingebettet. Das Strukturelement 1 besteht aus vier Leiterschichten 13, die voneinander über elektrische Isolierungen 5 getrennt sind. Die elektrisch leitenden Segmente 13, die beispielsweise aus einem Karbonfaserkomposit bestehen, bilden zusammen mit den Isolierschichten 5 eine strukturelle Einheit, die die mechanischen Funktionen des Strukturelementes 1 übernehmen. Zusätzlich bilden die leitend ausgebildeten Schichten oder Segmente 13 an den Außenkanten oder Außenseiten die Kontaktflächen 3 aus, an denen über Kontakte 10, beispielsweise Schleifkontakte, Schraubkontakte oder mit Spitzen versehene andere Kontakteinrichtungen das elektrische Signal abgenommen und weitergeleitet werden kann. In dem dargestellten Ausführungsbeispiel sind insgesamt vier Schleifkontakte 10 jeweils an den Schmalseiten des Strukturelementes angeordnet, es besteht auch die Möglichkeit, das elektrische Signal an der Stirnseite oder an den beiden Breitseiten des Strukturelementes 1 abzunehmen. Auch bei dieser Ausführungsform kann eine einfache Kürzung im Endbereich A stattfinden. Zweckmäßigerweise ist das gesamte Strukturelement 1 bei einer Ausgestaltung von Segmenten als Leiter 13 mit einer elektrischen Isolierung versehen, die nur teilweise an den Kontakten 10 durchbrochen oder aufgehoben wird, wenn das Strukturelement in die korrespondierende Aufnahmeeinrichtung eingeführt oder darin befestigt wird.

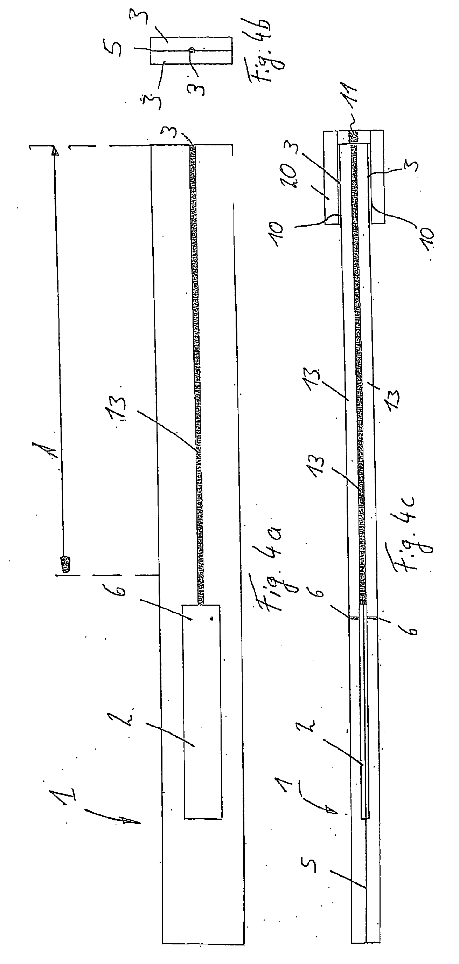

In der Figur 4 ist eine weitere Variante mit einem integrierten Sensor 2, einem Lichtleiter 13 und elektrisch leitenden Segmenten 13 vorgesehen, die durch eine Isolierschicht 5 voneinander getrennt sind. Die Figur 4a zeigt eine schematische Draufsicht auf die Variante, die Figur 4b eine stirnseitige Draufsicht und die Figur 4c eine schematische Schnittansicht des Strukturelementes 1 mit integriertem Sensor 2. Wie in den Figuren 4b und 4c zu erkennen ist, ist eine Isolierschicht 5 zwischen zwei Leiterschichten 13 vorhanden. Die Leiterschichten 13 sind beispielsweise aus einem Karbonkomposit oder einem anderen leitenden Material hergestellt. Zwischen den beiden Leiterschichten 13 ist ein Lichtleiter eingebettet, der mit dem Sensor 2 in Verbindung steht. Die Leiterelemente 13 sind mit dem Sensor 2 über Kontaktstifte 6 verbunden. Alternativ zu einer vollleitenden Ausgestaltung der Leiterelemente 13 kann auch nur die Oberfläche des Strukturelementes im Bereich des Endbereiches A elektrisch leitend ausgebildet sein und dort die Kontaktflächen bilden.

Der Lichtleiter 13 endet stirnseitig in einer Kontaktfläche 3, zu der korrespondierend ein Lichtleitersensor 11 an einer Aufnahmeeinrichtung 20 angeordnet ist. Die über den Lichtleiter 13 und die Kontaktfläche 3 übertragenden Lichtsignale werden über den Lichtleitersensor 11 aufgenommen und weitergeleitet oder ggf. in elektrische Signale umgewandelt.

An der Aufnahmeeinrichtung 20, in der das Strukturelement 1 eingeführt ist, sind Kontaktflächen 10 vorgesehen, die in Kontakt mit den Kontaktflächen 3 auf der Außenseite des Endbereiches A treten. Die Kontaktflächen 10 der Aufnahmeeinrichtung 20 bewirken eine großflächige Auflage auf die einander gegenüberliegenden Außenseiten des Strukturelementes 1. Auch hier können die Kontaktflächen 3 zunächst isoliert ausgebildet sein und erst zum Zweck der Montage abisoliert werden.

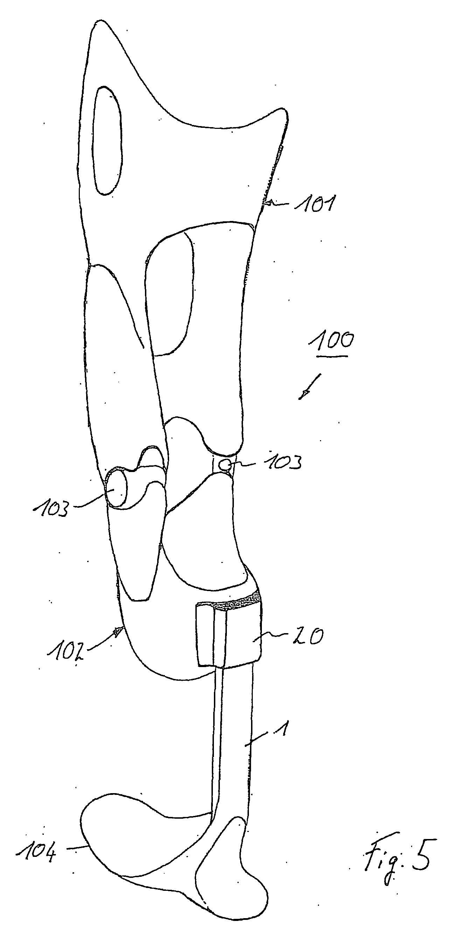

In der Figur 5 ist eine orthopädietechnische Einrichtung 100 in Gestalt einer Beinorthese dargestellt. Die Beinorthese 100 weist eine Aufnahmeeinrichtung 101 für einen Oberschenkel sowie eine Aufnahmeeinrichtung 102 für einen Unterschenkel auf. Beide Aufnahmeeinrichtungen 101, 102 sind über Gelenkeinrichtungen 103 im Bereich der natürlichen Knieachse schwenkbar miteinander verbunden. An der Aufnahmeinrichtung 102 für den Unterschenkel ist eine Aufnahme 20 angeordnet, die ein Strukturelement 1 in Gestalt einer Unterschenkelschiene eingesteckt ist. An dem distalen Ende des Strukturelementes 1 ist eine Fußauflagen 104 angeformt, auf die der Patientenfuß gestellt werden kann. Die Aufnahmeeinrichtung 101, 102 können aus Kunststoff oder einem Verbundwerkstoff ausgebildet sein. Die Aufnahme 20 kann einstückig an der Aufnahmeeinrichtung 102 für den Unterschenkel ausgebildet oder nachträglich daran befestigt sein. Das Strukturelement 1 ist in Gestalt eines Balkens ausgebildet und weist eine nicht näher dargestellte Sensoreinrichtung auf, die mit Leitern verbunden ist, die mit dem Strukturelement 1 integriert sind. Die Sensorsignale werden dann über ebenfalls nicht dargestellte Kontakte innerhalb der Aufnahme 20 aufgenommen und an eine Auswerteeinheit weitergeleitet.

Um die Orthese 100 nicht individuell anfertigen zu müssen, können die Aufnahmeeinrichtungen 101, 102 im Wesentlichen vorgefertigt ausgebildet werden. Die Oberschenkaufnahmeeinrichtung 101 kann an dem proximalen Ende entweder gekürzt werden oder ist so kurz ausgebildet, dass auch Patienten mit sehr kurzen Oberschenkeln damit ausgerüstet werden können. Selbst dann ist die Oberschenkelaufnahme 101 in der Regel lang genug, um eine sichere Festlegung auch bei großen Patienten zu gewährleisten.

Um auch eine Längenanpassung zwischen der Fußstütze 104 und der Gelenkeinrichtung 103 zu ermöglichen, damit die Orthese 100 korrekt an dem Patienten angepasst werden kann, ist das Strukturelement 1 kürzbar ausgebildet, so dass der für die Funktion der Orthese wichtige Abstand zwischen der Fußstütze 104 und der Gelenkachse 103 individuell angepasst werden kann, ohne dass aufwändige Umarbeiten bei der elektrischen Kontaktierung erfolgen müssen. Das Strukturelement 1 ist in der vorliegenden Ausführungsform in Richtung hinten angeordnet und kann federnde Eigenschaften aufweisen, grundsätzlich ist es jedoch auch möglich, dass die Sensoren nicht in dem balkenartigen Teil des Strukturelementes 1 angeordnet sind, sondern im Bereich der Fußstütze 104. Auch ist es möglich, dass die Fußstütze 104 separat ausgebildet und an dem Strukturelement 1 befestigt sein kann.

Neben Orthesen können die Strukturelemente 1 auch in Prothesen eingesetzt werden, der Anwendungszweck ist nicht auf Orthesen oder Prothesen für untere Extremitäten begrenzt, vielmehr können die Strukturelemente 1 überall dort eingesetzt werden, wo eine Längenanpassbarkeit einer orthopädietechnischen Einrichtung notwendig ist und Sensordaten aus der orthopädietechnischen Einrichtung erfasst und abgeleitet werden sollen.

## Patentansprüche

1. Strukturelement für eine orthopädietechnische Einrichtung, das zumindest einen in oder an dem Strukturelement (1) angeordneten Sensor (2) aufweist, der mit Leitern (13) zur Übertragung von Energie und/oder Sensorsignalen verbunden ist, **dadurch gekennzeichnet, dass** die Leiter (13) in dem Strukturelement (1) integriert sind, sich entlang eines Endbereiches (A) des Strukturelementes (1) erstrecken, in dem Endbereich (A) münden und dort Kontaktflächen (3) ausbilden.

2. Strukturelement nach Anspruch 1, **dadurch gekennzeichnet, dass** der Sensor (2) vollständig in dem Strukturelement (1) eingebettet ist.

3. Strukturelement nach Anspruch 1 oder 2, **dadurch gekennzeichnet, dass** der Sensor (2) als Dehnmessstreifen, optischer Dehnmessstreifen oder Piezoelement ausgebildet ist.

4. Strukturelement nach einem der voranstehenden Ansprüche, **dadurch gekennzeichnet, dass** das Strukturelement (1) als Federelement ausgebildet ist.

5. Strukturelement nach einem der voranstehenden Ansprüche, **dadurch gekennzeichnet, dass** die Leiter (13) innerhalb des Strukturelements (1) und die Kontaktflächen (3) in dem Endbereich (A) gegeneinander isoliert sind.

6. Strukturelement nach einem der voranstehenden Ansprüche, **dadurch gekennzeichnet, dass** die Kontaktflächen (3) im Endbereich (A) als Schleifkontakte oder Kontaktbahnen ausgeführt sind, die sich an zumindest einer Außenseite des Endbereiches (A) erstrecken.

7. Strukturelement nach einem der voranstehenden Ansprüche, **dadurch gekennzeichnet, dass** die Leiter (13) stirnseitig aus dem Strukturelement (1) herausgeführt sind und dort münden.

8. Strukturelement nach einem der voranstehenden Ansprüche, **dadurch gekennzeichnet, dass** die Leiter (13) rohrförmig ausgebildet sind.

9. Strukturelement nach einem der voranstehenden Ansprüche, **dadurch gekennzeichnet, dass** das Strukturelement (1) zumindest bereichsweise segmentiert ist und die Segmente gegeneinander isoliert und als Leiter ausgebildet sind.

10. Strukturelement nach einem der voranstehenden Ansprüche, **dadurch gekennzeichnet, dass** das Strukturelement (1) an seinem Umfang elektrisch isoliert ist.

11. Strukturelement nach einem der voranstehenden Ansprüche, **dadurch gekennzeichnet, dass** das Strukturelement (1) zumindest teilweise einen eckigen Querschnitt aufweist.

12. Strukturelement nach einem der voranstehenden Ansprüche, **dadurch gekennzeichnet, dass** das Strukturelement (1) aus einem Karbonfaserkomposit und/oder einem Glasfaserkomposit und/oder einem Kunststoff hergestellt ist.

13. Strukturelement nach einem der voranstehenden Ansprüche, **dadurch gekennzeichnet, dass** das Strukturelement (1) kürzbar ausgebildet ist.

14. Strukturelement nach einem der voranstehenden Ansprüche, **dadurch gekennzeichnet, dass** der Leiter als Lichtleiter ausgebildet ist.

15. Strukturelement nach einem der voranstehenden Ansprüche, **dadurch gekennzeichnet, dass** der Sensor (2) als eine Lichtquelle mit zumindest einem daran gekoppelten Lichtleiter ausgebildet ist, wobei der Lichtleiter das Lichtsignal der Lichtquelle in Abhängigkeit von der Belastung des Strukturelementes (1) verändert.

16. Orthopädische Einrichtung, insbesondere Orthese oder Prothese, mit einem Oberteil und einem Unterteil, die über ein Strukturelement (1) nach einem der voranstehenden Ansprüche miteinander verbunden sind.

17. Orthopädische Einrichtung nach Anspruch 16, **dadurch gekennzeichnet, dass** in dem Oberteil und/oder Unterteil Aufnahmeeinrichtungen (20) zur Festlegung des Strukturelementes (1) angeordnet sind, in denen Kontakte (10) angeordnet sind, die korrespondierend zu den Kontaktflächen (3) des Strukturelementes (1) im montierten Zustand ausgebildet sind.

18. Orthopädische Einrichtung nach Anspruch 16 oder 17, **dadurch gekennzeichnet, dass** die Kontakte (10) und/oder die Kontaktflächen (3) in Richtung aufeinander federbelastet und verlagerbar ausgebildet sind.

19. Orthopädische Einrichtung nach Anspruch 16 oder 17, **dadurch gekennzeichnet, dass** die Kontakte (10) als Steckkontakte, Schraubkontakte, Klemmeinrichtungen oder mit einer eine Isolierung durchdringenden Spitze ausgebildet sind.

## Claims

1. Structural element for an orthopedic device, having at least one sensor (2) arranged in or on the structural element (1), which sensor is connected to conductors (13) for transmitting energy and/or sensor signals, **characterized in that** the conductors (13) are integrated into the structural element (1), extend along an end region (A) of the structural element (1), open out into the end region (A) and form contact surfaces (3) there.

2. Structural element as claimed in claim 1, **characterized in that** the sensor (2) is completely embedded into the structural element (1).

3. Structural element as claimed in claim 1 or 2, **characterized in that** the sensor (2) is embodied as a strain gauge, optical strain gauge or piezoelement.

4. Structural element as claimed in one of the preceding claims, **characterized in that** the structural element (1) is embodied as a spring element.

5. Structural element as claimed in one of the preceding claims, **characterized in that** the conductors (13) within the structural element (1) and the contact surfaces (3) in the end region (A) are insulated from one another.

6. Structural element as claimed in one of the preceding claims, **characterized in that** the contact surfaces (3) in the end region (A) are embodied as sliding contacts or contact tracks, which extend along at least one outer side of the end region (A).

7. Structural element as claimed in one of the preceding claims, **characterized in that** the conductors (13) are routed out of the structural element (1) at the end face and open out there.

8. Structural element as claimed in one of the preceding claims, **characterized in that** the conductors (13) have a tubular design.

9. Structural element as claimed in one of the preceding claims, **characterized in that** the structural element (1) is segmented, at least in regions, and the segments are insulated from one another and embodied as conductors.

10. Structural element as claimed in one of the preceding claims, **characterized in that** the structural element (1) is electrically insulated around the circumference thereof.

11. Structural element as claimed in one of the preceding claims, **characterized in that** the structural element (1), at least in part, has an angular cross section.

12. Structural element as claimed in one of the preceding claims, **characterized in that** the structural element (1) is produced from a carbon-fiber composite and/or a glass-fiber composite and/or a plastic.

13. Structural element as claimed in one of the preceding claims, **characterized in that** the structural element (1) is embodied such that it can be shortened.

14. Structural element as claimed in one of the preceding claims, **characterized in that** the conductor is embodied as an optical fiber.

15. Structural element as claimed in one of the preceding claims, **characterized in that** the sensor (2) is embodied as a light source with at least one optical fiber coupled thereto, wherein the optical fiber modifies the light signal from the light source dependent on the load on the structural element (1).

16. Orthopedic device, more particularly an orthosis or a prosthesis, with an upper part and a lower part, which are interconnected via a structural element (1) as claimed in one of the preceding claims.

17. Orthopedic device as claimed in claim 16, **characterized in that** receptacle devices (20) for fixing the structural element (1) are arranged in the upper part and/or lower part, in which receptacle devices contacts (10) are arranged, which are embodied in a corresponding fashion to the contact surfaces (3) of the structural element (1) in the assembled state.

18. Orthopedic device as claimed in claim 16 or 17, **characterized in that** the contacts (10) and/or the contact surfaces (3) are embodied in a springloaded and displaceable fashion in the direction toward one another.

19. The orthopedic device as claimed in claim 16 or 17, **characterized in that** the contacts (10) are embodied as plug-in contacts, screw contacts, clamping apparatuses or with a tip that pierces the insulation.

## Revendications

1. Élément de structure pour dispositif orthopédique, lequel élément présente au moins un capteur (2) disposé dans ou sur l'élément de structure (1) et relié à des conducteurs (13) pour la transmission d'énergie et/ou de signaux de capteur, **caractérisé en ce que** les conducteurs (13) sont intégrés dans l'élément de structure (1), s'étendent le long d'une région terminale (A) de l'élément de structure (1), débouchent dans la région terminale (A) et y forment des surfaces de contact (3).

2. Élément de structure selon la revendication 1, **caractérisé en ce que** le capteur (2) est entièrement encastré dans l'élément de structure (1).

3. Élément de structure selon la revendication 1 ou 2, **caractérisé en ce que** le capteur (2) est réalisé sous forme de jauge d'allongement, de jauge d'allongement optique ou de piézo-élément.

4. Élément de structure selon l'une des revendications précédentes, **caractérisé en ce que** l'élément de structure (1) est réalisé sous forme d'élément ressort.

5. Élément de structure selon l'une des revendications précédentes, **caractérisé en ce que** les conducteurs (13) à l'intérieur de l'élément de structure (1) et les surfaces de contact (3) dans la zone terminale (A) sont mutuellement isolés.

6. Élément de structure selon l'une des revendications précédentes, **caractérisé en ce que** dans la zone terminale (A) les surfaces de contact (3) sont réalisées sous forme de contacts glissants ou de pistes de contact qui s'étendent sur au moins un côté extérieur de la zone terminale (A).

7. Élément de structure selon l'une des revendications précédentes, **caractérisé en ce que** les conducteurs (13) sont ramenés à l'extérieur de l'élément de structure (1) en extrémité, et y débouchent.

8. Élément de structure selon l'une des revendications précédentes, **caractérisé en ce que** les conducteurs (13) sont réalisés tubulaires.

9. Élément de structure selon l'une des revendications précédentes, **caractérisé en ce que** l'élément de structure (1) est segmenté au moins par zones et les segments sont mutuellement isolés et réalisés sous forme de conducteurs.

10. Élément de structure selon l'une des revendications précédentes, **caractérisé en ce que** l'élément de structure (1) est isolé électriquement sur son pourtour.

11. Élément de structure selon l'une des revendications précédentes, **caractérisé en ce que** l'élément de structure (1) présente au moins en partie une section transversale anguleuse.

12. Élément de structure selon l'une des revendications précédentes, **caractérisé en ce que** l'élément de structure (1) est fabriqué en un composite de fibres de carbone et/ou un composite de fibres de verre et/ou une matière synthétique.

13. Élément de structure selon l'une des revendications précédentes, **caractérisé en ce que** l'élément de structure (1) est réalisé raccourcissable.

14. Élément de structure selon l'une des revendications précédentes, **caractérisé en ce que** le conducteur est réalisé sous forme de conducteur de lumière.

15. Élément de structure selon l'une des revendications précédentes, **caractérisé en ce que** le capteur (2) est réalisé sous forme d'une source lumineuse associée à au moins un conducteur de lumière couplé à celle-ci, le conducteur de lumière modifiant le signal lumineux de la source lumineuse en fonction de la sollicitation subie par l'élément de structure (1).

16. Dispositif orthopédique, en particulier orthèse ou prothèse, comprenant une partie supérieure et une partie inférieure, reliées entre elles par l'intermédiaire d'un élément de structure (1) selon l'une des revendications précédentes.

17. Dispositif orthopédique selon la revendication 16, **caractérisé en ce que** des dispositifs de montage (20) sont agencés dans la partie supérieure et/ou dans la partie inférieure pour la fixation de l'élément de structure (1), dans lesquels sont agencés des contacts (10) qui sont réalisés pour correspondre aux surfaces de contact (3) de l'élément de structure (1) à l'état monté.

18. Dispositif orthopédique selon la revendication 16 ou 17, **caractérisé en ce que** les contacts (10) et/ou les surfaces de contact (3) sont réalisés pour être déplaçables et poussés élastiquement en direction l'un vers l'autre.

19. Dispositif orthopédique selon la revendication 16 ou 17, **caractérisé en ce que** les contacts (10) sont réalisés sous forme de contacts à fiche, de contact à vis, de dispositifs à serrage ou avec une pointe transperçant une isolation.
